(19) **Europäisches Patentamt / European Patent Office / Office européen des brevets**

(11) **EP 2 352 387 B1**

(12) **EUROPEAN PATENT SPECIFICATION**

(45) Date of publication and mention of the grant of the patent:
**06.09.2017 Bulletin 2017/36**

(21) Application number: **09761119.8**

(22) Date of filing: **23.11.2009**

(51) Int Cl.:
*A23K 20/20* (2016.01)    *A23K 50/40* (2016.01)
*A61K 31/00* (2006.01)    *A61K 33/04* (2006.01)
*A61K 33/06* (2006.01)    *A61K 33/42* (2006.01)
*A61K 45/06* (2006.01)    *A23L 33/16* (2016.01)

(86) International application number:
**PCT/US2009/065499**

(87) International publication number:
**WO 2010/060025 (27.05.2010 Gazette 2010/21)**

(54) **METHOD FOR MODIFYING FAT DIGESTIBILITY**

VERFAHREN ZUR MODIFIKATION DER VERDAULICHKEIT VON FETT

PROCÉDÉ POUR MODIFIER LA DIGESTIBILITÉ DES GRAISSES

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **21.11.2008 US 275544**

(43) Date of publication of application:
**10.08.2011 Bulletin 2011/32**

(73) Proprietor: **Hill's Pet Nutrition, Inc.**
**Topeka, KS 66603 (US)**

(72) Inventors:
- **YAMKA, Ryan, Michael**
**Topeka**
**Kansas 66618 (US)**
- **FRIESEN, Kim, Gene**
**Carthage**
**Indiana 46115 (US)**
- **KATS, Lauren**
**Topeka**
**Kansas 66614 (US)**
- **FORSTER, Thomas, Gordon**
**Topeka**
**Kansas 66614 (US)**

(74) Representative: **Wichmann, Hendrik et al**
**Wuesthoff & Wuesthoff**
**Patentanwälte PartG mbB**
**Schweigerstraße 2**
**81541 München (DE)**

(56) References cited:
**WO-A1-2007/065172**    **WO-A1-2007/082143**

- **MEYER H ET AL: "DIGESTIBILITY AND COMPATIBILITY OF MIXED DIETS AND FAECAL CONSISTENCY IN DIFFERENT BREEDS OF DOG" JOURNAL OF VETERINARY MEDICINE. SERIES A - ZENTRALBLATT FUERVETERINAERMEDIZIN. REIHE A, PAREY, BERLIN, DE, vol. 46, no. 3, 1 January 1999 (1999-01-01), pages 155-165, XP000992610 ISSN: 0931-184X**
- **DERSJANT-LI Y ET AL: "Feed intake, growth, digestibility of non dry matter and nitrogen in young pigs as affected by dietary cation-anion difference and supplementation of xylanase" JOURNAL OF ANIMAL PHYSIOLOGY AND ANIMAL NUTRITION, BLACKWELL, BERLIN, DE, vol. 85, no. 3-4, 1 April 2001 (2001-04-01), pages 101-109, XP002426462 ISSN: 0931-2439**
- **BAKER D H ET AL: "COMPARATIVE NUTRITION OF CATS AND DOGS" ANNUAL REVIEW OF NUTRITION, ANNUAL REVIEWS INC., PALO ALTO, CA, US, vol. 11, 1 July 1991 (1991-07-01), pages 239-264, XP008076850 ISSN: 0199-9885**

**Description**

CROSS-REFERENCE TO RELATED APPLICATION

This application is a continuation-in-part application of pending U.S. patent application number 11/566,512, which was filed December 4, 2006 and claims the benefit of U.S. Provisional Application Serial No. 60/741,632 filed December 2, 2005, and is also a continuation-in-part application of pending U.S. patent application number 11/620,119, which was filed January 5, 2007 and claims the benefit of U.S. Provisional Application Serial No. 60/765,341 filed January 5, 2006.

FIELD OF THE INVENTION

The invention encompasses methods for modifying fat digestibility in a dog by adjusting the balance of metabolizable cations to metabolizable anions consumed by the dog.

BACKGROUND OF THE INVENTION

Dietary cation anion balance (DCAB) may be manipulated to improve the overall quality of animal fodder and this has been of particular interest with regard to increasing feed efficiencies in the swine industry. (NRC, 1998, Nutrient Requirements of Swine, 10th Ed., National Academy Press, Washington, D.C.). It has been reported that a negative DCAB can reduce voluntary food intake in swine while diets with a positive DCAB can cause an increase in swine growth (Derjant-Li et al., 2001, J. Anim. Sci. 79:1840-1848). The precise DCAB for optimal performance varies in the literature, however, and may be due to the effect of particular feed components on DCAB. Conflicting data has also been reported with regard to positive and negative DCAB on nutrient digestibility in swine, with some studies suggesting that increasing DCAB values can increase dry matter, energy and nitrogen digestibility (Haydon and West, 1990, J. Anim. Sci 68:3687-3693;
Derjant-Li et al., 2001, J. Anim. Sci. 79:1840-1848) and others reporting improvements in feed efficiency and nitrogen digestibility with increasing dietary chloride levels, i.e. increasing negative DCAB. (Mahan et al. 1999, J. Anim. Sci. 77:3016-3021).

The inventors have discovered that manipulation of dietary cation-anion balance can improve stool quality and stool frequency in companion animals, improve nutrient digestibility in companion animals, and alter food intake in companion animals.

Meyer H. et al 1999, (J. Vet. Med. Vol. 46, pages 155 to 165; XP000992610) discloses a study investigating the digestibility and compatibility of mixed diets and faecal consistency in different breeds of dog.

Dersjant-Li et al, 2001 (J. Anim. Physiol. a. Anim. Nutr., vol 85, pages 101 to 109; XP002426462) discloses feed intake, growth, digestibility of dry matter and nitrogen in young pigs as affected by dietary cation-anion difference and supplementation of xylanase.

WO2007/065172 A1 discloses methods for altering stool quality and/or stool frequency.

SUMMARY OF THE INVENTION

In a first aspect, the present invention specifically provides metabolizable cations and metabolizable anions for use in increasing fat digestibility in a dog in need thereof, wherein the metabolizable cations and metabolizable anions are provided in a composition, wherein the dog has a previous diet, wherein the dietary cation-anion balance (DCAB) of said composition is increased relative to the dietary cation-anion balance (DCAB) of the previous diet of the dog, and wherein the composition is administered to the dog.

In a second aspect, the present invention specifically provides metabolizable cations and metabolizable anions for use in decreasing fat digestibility in a dog in need thereof, wherein the metabolizable cations and metabolizable anions are provided in a composition, wherein the dog has a previous diet, wherein the dietary cation-anion balance (DCAB) of said composition is decreased relative to the dietary cation-anion balance (DCAB) of the previous diet of the dog, and wherein the composition is administered to the dog.

In a third aspect, the present invention provides a use of metabolizable anions and metabolizable cations in the manufacture of a dog food composition to modify fat digestibility in a dog in need thereof, wherein the dog has a previous diet, wherein the dietary cation-anion balance (DCAB) of said composition is increased or decreased relative to the dietary cation-anion balance (DCAB) of the previous diet of the dog, wherein the composition is administered to the dog, and wherein a decrease in DCAB decreases fat digestibility, and an increase in DCAB increases fat digestibility in the dog.

The description generally encompasses compositions and methods with beneficial results for companion animals achieved by adjusting the balance of metabolizable cations to metabolizable anions in the compositions consumed by an animal by an amount sufficient to alter and therefore improve stool quality and/or stool frequency of an animal. The description also encompasses kits comprising combinations of cations, anions, foods, compounds, instructions, and devices useful for altering stool quality and/or stool frequency are also provided.

In one aspect, the description encompasses compositions including two or more ingredients that, when combined together and optionally with additional ingredients that are not a part of the composition, yield a composition for use in the invention and/or in the methods defined herein. The compositions include a food intake altering amount of at least one ingredient comprising a metabolizable cation or metabolizable anion and at least one of (1) an ingredient comprising a different metabolizable cation or metabolizable anion; (2) one or more ingredients for consumption by an animal; (3) one or more weight-loss agents; and (4) one or more agents for promoting weight-gain.

In another aspect, the description encompasses methods for improving stool quality for an animal. In another aspect, the description encompasses methods for altering stool frequency of an animal.

In another aspect, the description encompasses methods for modifying nutrient digestibility in a dog in need thereof. In certain aspects, the description encompasses methods for increasing protein digestibility in a dog in need thereof comprising administering to the dog a composition, wherein the composition has a decreased DCAB. In one aspect, a dog in need thereof is a dog suffering from irritable bowel disease (IBD). In another aspect, the description encompasses methods for decreasing protein digestibility in a dog in need thereof comprising administering to the dog a composition, wherein the composition has an increased DCAB. In another aspect, a dog in need thereof is a dog suffering from kidney disease.

As mentioned above, the invention encompasses using metabolizable cations and metabolizable anions in increasing fat digestibility in a dog in need thereof, wherein the metabolizable cations and metabolizable anions are provided in a composition, wherein the dog has a previous diet, wherein the dietary cation-anion balance (DCAB) of said composition is increased relative to the dietary cation-anion balance (DCAB) of the previous diet of the dog, and wherein the composition is administered to the dog. In one aspect, a dog in need thereof is a dog in which weight gain is desired. In another aspect, as mentioned above, the invention encompasses using metabolizable cations and metabolizable anions in decreasing fat digestibility in a dog in need thereof, wherein the metabolizable cations and metabolizable anions are provided in a composition, wherein the dog has a previous diet, wherein the dietary cation-anion balance (DCAB) of said composition is decreased relative to the dietary cation-anion balance (DCAB) of the previous diet of the dog, and wherein the composition is administered to the dog. In one aspect, a dog in need thereof is a dog in which weight reduction is desired.

In another aspect, the description encompasses methods for treating a dog suffering from digestive problems comprising adjusting the balance of metabolizable cations to metabolizable anions consumed by the dog by an amount sufficient to modify the digestibility of fat and/or protein in said dog and wherein said modification provides a beneficial therapeutic effect. In one aspect, the balance of metabolizable cations to metabolizable anions consumed by the dog is decreased in order to increase protein digestibility and/or decrease fat digestibility in the dog. In the present invention, the balance of metabolizable cations to metabolizable anions consumed by the dog is decreased in order to decrease fat digestibility. According to this method, the balance of metabolizable cations to metabolizable anions may be decreased by decreasing the amount of metabolizable cations consumed by the dog, increasing the amount of metabolizable anions consumed by the dog or by both decreasing the amount of metabolizable cations and increasing the amount of metabolizable anions consumed by the dog.

In another aspect, the description encompasses methods for increasing the balance of metabolizable cations to metabolizable anions consumed by the dog in order to decrease protein digestibility and/or increase fat digestibility in the dog. In the present invention, the balance of metabolizable cations to metabolizable anions consumed by the dog is increased in order to increase fat digestibility. According to this method, the balance of metabolizable cations to metabolizable anions may be increased by increasing the amount of metabolizable cations consumed by the dog, decreasing the amount of metabolizable anions consumed by the dog or by both increasing the amount of metabolizable cations and decreasing the amount of metabolizable anions consumed by the dog.

In another aspect, the description encompasses methods for supplementing a dog's diet with protein or fat to minimize the effects of any undesired inverse changes in protein or fat digestibility.

In another aspect, the description encompasses methods for altering average food intake by an animal. The methods include adjusting the balance of metabolizable cations to metabolizable anions consumed by the animal by an amount effective to alter average food intake.

In another aspect, the description provides encompasses methods for controlling weight of an animal. The methods include adjusting the balance of metabolizable cations to metabolizable anions consumed by the animal to alter average food intake in an amount effective to influence the animal's weight.

In another aspect, the description provides kits suitable for altering and improving stool quality and/or stool frequency.

In another aspect, the description provides means for communicating information about the methods and kits described herein and their use for altering and improving stool quality and/or stool frequency.

## DETAILED DESCRIPTION OF THE INVENTION

General Description

The description provides methods for increasing protein digestibility in a dog in need thereof comprising administering to the dog a composition, wherein the composition has a decreased dietary cation anion balance (DCAB).
In certain aspects, the dog is suffering from irritable bowel disease (IBD).
The description also provides methods for decreasing protein digestibility in a dog in need thereof comprising administering to the dog a composition, wherein the composition has an increased DCAB.
In certain aspects, the dog is suffering from kidney disease.
The invention also encompasses metabolizable cations and metabolizable anions for use in increasing fat digestibility in a dog in need thereof, wherein the metabolizable cations and metabolizable anions are provided in a composition, wherein the dog has a previous diet, wherein the dietary cation-anion balance (DCAB) of said composition is increased relative to the dietary cation-anion balance (DCAB) of the previous diet of the dog, and wherein the composition is administered to the dog.
In certain aspects, the dog is a dog in which weight gain is desired.
The invention also encompasses metabolizable cations and metabolizable anions for use in decreasing fat digestibility in a dog in need thereof, wherein the metabolizable cations and metabolizable anions are provided in a composition, wherein the dog has a previous diet, wherein the dietary cation-anion balance (DCAB) of said composition is decreased relative to the dietary cation-anion balance (DCAB) of the previous diet of the dog, and wherein the composition is administered to the dog.
In certain aspects, the dog is a dog in which weight reduction is desired.
In the invention, the uses defined herein further comprise treating a dog suffering from digestive problems, wherein the uses comprise adjusting the balance of metabolizable cations to metabolizable anions consumed by the dog by an amount sufficient to modify the digestibility of fat in said dog and wherein said modification provides a beneficial therapeutic effect.
In certain embodiments, the balance of metabolizable cations to metabolizable anions consumed by the dog is decreased in order to decrease fat digestibility in the dog.
In certain embodiments, the balance of metabolizable cations to metabolizable anions may be decreased by decreasing the amount of metabolizable cations consumed by the dog, increasing the amount of metabolizable anions consumed by the dog or by both decreasing the amount of metabolizable cations and increasing the amount of metabolizable anions consumed by the dog.
In certain embodiments, the balance of metabolizable cations to metabolizable anions consumed by the dog is increased in order to increase fat digestibility in the dog.
In certain embodiments, the balance of metabolizable cations to metabolizable anions may be increased by increasing the amount of metabolizable cations consumed by the dog, decreasing the amount of metabolizable anions consumed by the dog or by both increasing the amount of metabolizable cations and decreasing the amount of metabolizable anions consumed by the dog.
In certain aspects, the description provides supplementation of the dog's diet with protein or fat to minimize the effects of any undesired inverse changes in protein or fat digestibility.
The invention also encompasses a use of metabolizable anions and metabolizable cations in the manufacture of a dog food composition to modify fat digestibility in a dog in need thereof, wherein the dog has a previous diet, wherein the dietary cation-anion balance (DCAB) of said composition is increased or decreased relative to the dietary cation-anion balance (DCAB) of the previous diet of the dog, wherein the composition is administered to the dog, and wherein a decrease in DCAB decreases fat digestibility, and an increase in DCAB increases fat digestibility in the dog.

Definitions

As used herein and in the appended claims, the singular forms "a", "an", and "the" include plural reference unless the context clearly indicates otherwise. The terms "comprise", "comprises", and "comprising" are to be interpreted inclusively rather than exclusively.
The term "animal" means any animal susceptible to or suffering from poor stool quality and/or irregular stool frequency. An animal is "susceptible to" a disease or condition if the animal exhibits symptoms that indicate that the animal is likely to develop the condition or disease. An animal is "suffering from" a disease or condition if the animal exhibits symptoms that are indicative that the animal has developed the condition or disease.
A "beneficial therapeutic effect" as used herein refers to a desirable change in a dog's physical well being which may be discerned by one of skill in the art.

A "decrease in DCAB" includes changes from a greater positive DCAB to a smaller positive DCAB, from a positive DCAB to a negative DCAB as well as a change from a smaller negative DCAB to a larger negative DCAB. All reflect changes to a more negative DCAB. Similarly, an "increase in DCAB" includes changes from a greater negative DCAB to a smaller negative DCAB, from a negative DCAB to a positive DCAB as well as a change from a smaller positive DCAB to a larger positive DCAB. All reflect changes to a greater positive DCAB.

As used herein, a "dog in need thereof includes any dog in which an increase or decrease in fat and/or protein digestibility is therapeutically beneficial including, but not limited to, dogs with digestive problems. For example, dogs in which an increase in protein digestibility is therapeutically beneficial include dogs suffering from irritable bowel disease (IBD), but also include dogs in which a reduction in stool odor is desired. Dogs in which a decrease in protein digestibility may be therapeutically beneficial include dogs suffering from kidney disease. Dogs in which an increase in fat digestibility may be desirable include dogs in which weight gain may be therapeutically beneficial, including, but not limited to, senior or geriatric dogs, dogs with cancer or other underweight dogs. Dogs in which a decrease in fat digestibility may be desirable include dogs in which weight loss is therapeutically beneficial.

As used herein, the term "digestive problems", as in, for example, "a dog suffering from digestive problems" refers to those conditions in which the digestion of protein and/or fat in a dog is abnormal. These conditions include conditions characterized by malabsorption of nutrients, which may be caused by, for example, digestive enzyme deficiencies, pancreatic insufficiency, inflammatory diseases, food allergies, diabetes mellitus, hypoadrenocorticism, ulcer, bacterial enteritis or colitis, or irritable bowel syndromes. Generally, an increase in fat and/or protein digestibility is desired in an animal suffering from a digestive problem, however, depending on the particular condition, one of skill in the art would be familiar with regard to whether an increase or decrease in fat and/or protein digestibility would cause a therapeutic benefit in a dog.

As used herein, a "greater negative DCAB" or a "greater positive DCAB" refers to a comparison with another DCAB value.

As used herein, "having a base excess" refers to a negative DCAB value, while "lacking a base excess" refers to a positive DCAB value. Thus, an increase in base excess refers to a greater negative DCAB value in comparison to another DCAB value. Similarly, a decrease in base excess refers to a greater positive DCAB value in comparison to another DCAB value.

As used herein, "modifying nutrient digestibility" refers to altering the break down and absorption of a nutrient in the body, e.g., either increasing or decreasing nutrient digestibility in a dog, for example, fat or protein digestibility. As used herein, "increasing digestibility" in a dog refers to causing a beneficial increase in the digestion of a nutrient such as may be typical for a normal dog of comparable breed, age and size, or otherwise as therapeutically desired in the dog. Similarly, as used herein, "decreasing digestibility" in a dog refers to causing a beneficial decrease in the digestion of a nutrient to levels typical for a normal dog of comparable breed, age and size, or otherwise as therapeutically desired in the dog. A modification in digestibility may be quantitated using conventional methods by comparing nutrient digestibility in a dog before and after treatment according to the methods of the present invention.

A dog is "suffering from" a disease or condition if the dog exhibits at least one symptom that is indicative that the dog has developed the condition or disease.

The metabolic acid-base status of an animal is influenced by the dietary cation (positively charged minerals) and anion (negatively charged minerals) balance consumed by an animal ("DCAB"). DCAB is measured as mEq.

As used herein, the term "undesired inverse changes" refers to a change in protein or fat digestibility in a dog due to increasing or decreasing the DCAB of the composition consumed by the dog that could have a negative effect on the health of the dog. An undesirable change in digestibility of fat or protein in a dog would be easily recognizable to one of skill in the art and could be lessened by supplementing the diet with protein or fat as disclosed herein.

Compositions of the Invention

The description provides compositions including metabolizable anions and metabolizable cations and the use of metabolizable anions and metabolizable cations in the manufacture of a dog food composition to modify protein and/or fat digestibility in a dog in need thereof, wherein a decrease in DCAB can increase protein digestibility and decrease fat digestibility and an increase in DCAB can decrease protein digestibility and increase fat digestibility in a dog in need thereof. Accordingly, the compositions also have utility in altering food intake and altering stool quality and/or stool frequency.

The cations and anions useful in the methods and compositions defined herein are any cation or anion suitable for consumption by an animal. In one aspect, the metabolizable cations are selected from the group consisting of calcium, sodium, potassium, and magnesium and the metabolizable anions are selected from the group consisting of phosphorus, chloride, and sulfur.

The balance of metabolizable cations to metabolizable anions in the compositions described herein can be determined by any means known to one of ordinary skill in the art. For example, one method for measuring the balance of metabolizable cations to metabolizable anions in a composition is to calculate the animal's dietary cation-anion balance (DCAB), which is determined by calculating the cumulative amount of sodium, potassium, calcium, and magnesium regularly consumed by the animal and subtracting the cumulative amount of chloride, sulfur, and phosphorus regularly consumed by the animal. (See, e.g., Baker ef a/., Comparative Nutrition of Cats and Dogs, Ann. Rev. Nutr. 11:239-63 (1991)).

In adjusting the balance of metabolizable cations to metabolizable anions in the compositions described herein, in certain aspects increasing the balance of metabolizable cations to metabolizable anions will result in firmer stool quality and reduced stool output. Conversely, decreasing the balance of metabolizable cations to metabolizable anions will result in looser stool and increased stool output.

As contemplated herein, the compositions described herein encompass nutritionally complete pet food compositions. Nutritionally complete pet food compositions, including nutritionally complete dog foods, are familiar to those of ordinary skill in the art. For example, nutrients and ingredients suitable for dog food compositions, and recommended amounts thereof, may be found in the Official Publication of the Association of American Feed Control Officials, Inc. ("AAFCO"), Nutrient Requirements of Dogs and Cats, 2006. Nutritionally complete foods may contain protein, fat, carbohydrate, fiber, amino acids, minerals, vitamins, and other ingredients at recommended levels and ratios known by those of skill in the art.

Protein may be supplied by any of a variety of sources known by those skilled in the art, including plant sources, animal sources, or both. Animal sources include, for example, meat, meat by products, seafood, dairy, eggs, etc. Meats include, for example, the flesh of poultry, fish, and mammals (e.g., cattle, pigs, sheep, goats, and the like). Meat by products include, for example, lungs, kidneys, brain, livers, and stomachs and intestines (freed of all or essentially all their contents). The protein can be, e.g., intact, almost completely hydrolyzed, or partially hydrolyzed.

Fat can be supplied by any of a variety of sources known by those skilled in the art, including meat, meat by-products, fish oil, and plants. Plant fat sources include wheat, flaxseed, rye, barley, rice, sorghum, corn, oats, millet, wheat germ, corn germ, soybeans, peanuts, and cottonseed, as well as oils derived from these and other plant fat sources.

Carbohydrate may be supplied by any of a variety of sources known by those skilled in the art, including oat fiber, cellulose, peanut hulls, beet pulp, parboiled rice, corn starch, corn gluten meal, and any combination of those sources. Grains supplying carbohydrate include, but are not limited to, wheat, corn, barley, and rice.

Fatty acids for inclusion in pet foods include omega 3 fatty acids such as docosahexanenoic acid (DHA), eicosapentaenoic acid (EPA), alpha-linolenic acid (ALA), octadecatetraenoic acid (stearidonic acid) or mixtures thereof.

Dietary fiber includes components of a plant, which are resistant to digestion by an animal's digestive enzymes. Dietary fiber includes soluble and insoluble fibers. Soluble fiber are resistant to digestion and absorption in the small intestine and undergo complete or partial fermentation in the large intestine, e.g., beet pulp, guar gum, chicory root, psyllium, pectin, blueberry, cranberry, squash, apples, oats, beans, citrus, barley, or peas. Insoluble fiber may be supplied by any of a variety of sources, including cellulose, whole wheat products, wheat oat, corn bran, flax seed, grapes, celery, green beans, cauliflower, potato skins, fruit skins, vegetable skins, peanut hulls, and soy fiber. Crude fiber includes indigestible components contained in cell walls and cell contents of plants such as grains, e.g., hulls of grains such as rice, corn, and beans.

Amino acids, including essential amino acids, may be added to pet food compositions as free amino acids, or supplied by any number of sources, e.g., crude protein. Essential amino acids are amino acids that cannot be synthesized de novo, or in sufficient quantities by an organism and thus must be supplied in the diet. Essential amino acids vary from species to species, depending upon the organism's metabolism. For example, it is generally understood that the essential amino acids for dogs and cats (and humans) are phenylalanine, leucine, methionine, lysine, isoleucine, valine, threonine, tryptophan, histidine and arginine. In addition, taurine, while technically not an amino acid but a derivative of cysteine, is an essential nutrient for cats.

Nutritionally complete pet food compositions may also contain one or more minerals and/or trace elements, e.g., calcium, phosphorus, sodium, potassium, magnesium, manganese, copper, zinc, or iron salts, in amounts required to avoid deficiency and maintain health. These amounts are known by those of skill in the art, for example, as provided by AAFCO guidelines.

Nutritionally complete pet food compositions may also include vitamins in amounts required to avoid deficiency and maintain health. These amounts, and methods of measurement, are known by those skilled in the art. For example, AAFCO provides recommended amounts of such ingredients for dogs and cats. As contemplated herein, useful vitamins may include, but are not limited to, vitamin A, vitamin B1, vitamin B2, vitamin B6, vitamin B12, vitamin C, vitamin D, vitamin E, vitamin H (biotin), vitamin K, folic acid, inositol, niacin, choline, and pantothenic acid.

Pet food compositions of the invention may additionally include additives, stabilizers, fillers, thickeners, flavorants, palatability enhancers and colorants in amounts and combinations familiar to one of skill in the art.

In one embodiment, the compositions of the invention are provided as a food, e.g., a nutritionally complete pet food composition. In another embodiment, the food compositions may be in the form of a treat, snack, supplement, or partially or fully edible toy. Such items for consumption by a pet are known to those skilled in the art, and can include, for example, compositions that are given to a dog to eat during non meal time, for example, a dog biscuits and edible chew toys.

Foods of any consistency or moisture content may be used in the methods of the present invention, e.g., a dry, moist or semi-moist dog food composition. "Semi-moist" refers to a food composition containing from about 25 to 35 % moisture. "Moist" food refers to a food composition that has a moisture content of from about 60 to 90% or greater. "Dry" food refers to a food composition from about 3 to 11% moisture content and is often manufactured in the form of small bits or kibbles. Also contemplated herein are compositions that may comprise components of various consistency as well as components that may include more than one consistency, for example, soft, chewy meat-like particles as well as kibble having an outer cereal component and an inner cream component as described in, e.g., US Patent 6,517,877. The kibble may be dried and optionally coated with one or more topical coatings known by those skilled in the art, for example, flavors, fats, oils, powders, and the like.

In some aspects of the description, the balance of metabolizable cations to metabolizable anions consumed by the dog can be adjusted in conjunction with the administration of one or more compositions comprising a gastrointestinal tract-improving agent, which may help digestion. "Gastrointestinal tract-improving agents" include probiotics and prebiotics.

Prebiotics are nondigestible food ingredients that beneficially affect host health by selectively stimulating the growth and/or activity of bacteria in the colon. The prebiotic, fructooligosaccharide (FOS) is found naturally in many foods such as wheat, onions, bananas, honey, garlic, and leeks. FOS can also be isolated from chicory root or synthesized enzymatically from sucrose. FOS fermentation in the colon results in a large number of physiologic effects including increasing the numbers of bifidobacteria in the colon, increasing calcium absorption, increasing fecal weight, shortening of gastrointestinal transit time, and possibly lowering blood lipid levels. The increase in bifidobacteria has been assumed to benefit human health by producing compounds to inhibit potential pathogens, by reducing blood ammonia levels, and by producing vitamins and digestive enzymes. Probiotic bacteria such as Lactobacilli or Bifidobacteria are believed to positively affect the immune response by improving the intestinal microbial balance leading to enhanced antibody production and phagocytic (devouring or killing) activity of white blood cells. Bifidobacterium lactis could be an effective probiotic dietary supplement for enhancing some aspects of cellular immunity in the elderly.

Probiotics enhance systemic cellular immune responses and may be useful as a dietary supplement to boost natural immunity in otherwise healthy adults. Probiotics include many types of bacteria but generally are selected from four genera of bacteria: Lactobacilllus acidophillus, Bifidobacteria, Lactococcus, and Pediococcus. The amount of probiotics and prebiotics to be administered to the animal is determined by the skilled artisan based upon the type and nature of the probiotic and prebiotic and the type and nature of the animal, e.g., the age, weight, general health, sex, extent of microbial depletion, presence of harmful bacteria, and diet of the animal. Generally, probiotics are administered to the animal in amounts of from about one to about twenty billion colony forming units (CFUs) per day for the healthy maintenance of intestinal microflora, preferably from about 5 billion to about 10 billion live bacteria per day. Generally, prebiotics are administered in amounts sufficient to positively stimulate the healthy microflora in the gut and cause these "good" bacteria to reproduce. Typical amounts are from about one to about 10 grams per serving or from about 5 percent to about 40 percent of the recommended daily dietary fiber for an animal.

In some embodiments, the balance of metabolizable cations to metabolizable anions consumed by the animal can be adjusted in conjunction with the administration of one or more compositions comprising a gastrointestinal tract improving agent selected from the group consisting of probiotics and prebiotics,

The amount of probiotics and prebiotics to be administered to a dog may be determined by the skilled artisan based upon the type and nature of the probiotic and prebiotic and the type and nature of the dog, e.g., the age, weight, general health, sex, extent of microbial depletion, presence of harmful bacteria, and diet of the dog. Generally, probiotics are administered to a dog in amounts of from about one to about twenty billion colony forming units (CFUs) per day for the healthy maintenance of intestinal microflora, preferably from about 5 billion to about 10 billion live bacteria per day. Generally, prebiotics are administered in amounts sufficient to positively stimulate the healthy microflora in the gut and cause these "good" bacteria to reproduce. Typical amounts are from about one to about 10 grams per serving or from about 5 percent to about 40 percent of the recommended daily dietary fiber for a dog,

As contemplated herein, in certain aspects, in the compositions defined herein it is desirable to modify the DCAB to about -200 to about -100 to achieve an increase in protein digestibility or to modify a DCAB to about +70 to about +200 to achieve an increase in fat digestibility. As described in the Examples, DCAB values of -128 and +98 were effective to achieve an increase in protein and fat digestibility in dogs, respectively.

The balance of metabolizable cations to metabolizable anions can be determined by any means known to skilled artisans. For example, DCAB may be determined for a dog by calculating the cumulative amount of cations regularly consumed by the dog and subtracting the cumulative amount of anions consumed by the dog. Cations may include, e.g., sodium, potassium, calcium, and magnesium cations, or any other ion having a positive charge, including amino acids. Anions may include, e.g., chloride, sulfur, and phosphorus anions, or any other ion having a negative charge, including amino acids. Pharmaceutical salt forms of these cations and anions are also included herein. For example, the DCAB may be determined by calculating the cumulative amounts of sodium, potassium, calcium, and magnesium cations regularly consumed by the dog and subtracting the cumulative amount of chloride, sulfur, and phosphorus anions regularly consumed by the dog, e.g., according to the following equation:

$$DCAB \ (mEq) = (Na + K + Ca + Mg) - (Cl + S + P)$$

(Baker and Czarnecki-Maulden, 1991, Annu. Rev. Nutr. 11:239-263).

The protein digestibility is then calculated as follows:

(amount of protein consumed - protein content of stool) X 100%/amount of protein consumed

Similarly, the fat content of food may be determined according to conventional methods and the apparent fat digestibility determined as follows:

(amount of fat consumed – fat content of stool) X 100%/amount of fat consumed

The digestibility of other nutrients may be determined in a similar fashion. For example, in order to determine carbohydrate digestibility, one of skill in the art can determine the carbohydrate content of a composition, and the carbohydrate content of stool produced by feeding the dog the composition. Carbohydrate content may be calculated as nitrogen free extract ("NFE") which may be calculated as follows: NFE = 100% - moisture% - protein % - fat % - ash% - crude fiber %. The carbohydrate digestibility is then calculated by the following equation:

(amount of carbohydrate consumed - carbohydrate content of stool) X 100%/amount of carbohydrate consumed

Dry matter digestibility (DMD) is the amount of matter that is digested by a dog on a dry matter basis. Methods for determining DMD are known in the art. For example, the mass of food consumed on a dry matter basis may be determined, and the mass of stool (on a dry matter basis) resulting from consumption of the food may also be determined. DMD is then calculated as follows:

(mass of food consumed - mass of stool produced) X 100%/mass of food consumed

Energy digestibility may also be determined, e.g.,

(amount of energy consumed- energy content of stool) X 100%/amount of energy consumed

Determination of energy content in food and stool may be performed according to conventional methods familiar to one of skill in the art.

Fiber digestibility may be determined by determining the fiber content in the food and stool and employing the following equation:

(amount of fiber consumed - fiber content of stool) X 100%/amount of fiber consumed

Methods for Improving Stool Quality

Also described herein are methods of improving the stool quality or stool frequency of a companion animal.

In one aspect, the description encompasses methods for improving stool quality for an animal. In another, the description encompasses methods for altering stool frequency for an animal. The methods include adjusting the balance of metabolizable cations to metabolizable anions in the compositions of the invention consumed by the animal by an amount sufficient to alter the animal's stool quality and/or stool frequency,

The methods described herein also encompass administering compositions that include a stool quality adjusting amount of at least one ingredient selected from the group consisting of metabolizable cations and metabolizable anions. In another aspect, the description provides for the use of such a composition to prepare a medicament for altering stool quality or altering stool frequency. Generally, medicaments are prepared by admixing a compound or composition with excipients, buffers, binders, plasticizers, colorants, diluents, compressing agents, lubricants, flavorants, moistening agents, and other ingredients known to skilled artisans to be useful for producing medicaments and formulating medicaments that are suitable for administration to an animal.

The methods described herein are useful for a variety of human and non-human animals, including avian, bovine, canine, equine, feline, hicrine, murine, ovine, and porcine animals, and are particularly useful for companion animals such as canines and felines, including dogs and cats. In some embodiments, the animal is a member of the order *Carnivora.* In some embodiments, the animal is a canine, and in other such embodiments a feline. In some embodiments, the animal is a companion animal. A companion animal can be, for example, an animal of any species that is kept as a pet. A companion animal can also be an animal from a variety of widely domesticated species, for example, dogs *[Canis familiahs)* and cats {*Felis domesticus)* regardless of whether or not the animal is kept solely as a pet. Thus, companion animals include, for example, working dogs, cats kept for rodent control, as well as pet cats and dogs.

Stool quality may be determined according to conventional methods. For example, fecal quality is commonly assessed by those of skill in the art by visual scoring, e.g., ranking stool visually on a scale from grade 1-5 as follows:

Grade 1: Greater than two-thirds of the feces in a defecation are liquid. The feces have lost all form, appearing as a puddle or squirt.
Grade 2: Soft-liquid feces are an intermediate between soft and liquid feces. Approximately equal amounts of feces in a defecation are soft and liquid,
Grade 3: Greater than two-thirds of the feces in a defecation are soft. The feces retain enough form to pile but have lost their firm cylindrical appearance,
Grade 4: Firm-soft feces are an intermediate between the grades of firm and soft. Approximately equal amounts of feces in a defecation are firm and soft.
Grade 5: Greater than two-thirds of the feces in a defecation are firm, They have a cylindrical shape with little flattening.

See also, Sunvold et al., J Anim Sci 1995 73:1099-1109; US Patent 6,280,779; US Patent 5,616,569. Stool quality may also be assessed quantitatively using methods to determine the amount of moisture in the feces in g/kg (see, e.g., Yamka et al., 2006, Am J Vet Res, 67(1):88-94).

In some aspects wherein the animal is susceptible to or suffering from loose stool and/or frequent stool output, stool quality and stool frequency may be improved by increasing the balance of metabolizable cations to metabolizable anions to provide the animal with firmer stool and/or to reduce stool output. In such aspects, the balance of cations to anions can be increased by increasing the cumulative amount of calcium, sodium, potassium, and magnesium regularly consumed by the animal relative to the cumulative amount of phosphorus, chloride, and sulfur regularly consumed by the animal. For example, the balance of metabolizable cations to metabolizable anions may be increased by increasing the animal's dietary intake of at least one composition comprising a cation excess of calcium, sodium, potassium, or magnesium. Likewise, the balance may be increased by decreasing the animal's dietary intake of at least one composition comprising an anion excess of chloride, phosphorus or sulfur.

In other aspects wherein the animal is susceptible to or suffering from constipation, stool quality and/or stool frequency can be improved by decreasing the balance of metabolizable cations to metabolizable anions consumed by the animal to provide the animal with looser stool and/or increased stool output. In such aspects, the balance of cations to anions can be decreased by decreasing the cumulative amount of calcium, sodium, potassium, and magnesium regularly consumed by the animal relative to the cumulative amount of phosphorus, chloride, and sulfur regularly consumed by the animal. For example, the balance of metabolizable cations to metabolizable anions can be decreased by decreasing the animal's dietary intake of at least one composition comprising a cation excess of calcium, sodium, potassium, or magnesium. Likewise, the balance may be decreased by increasing the animal's dietary intake of at least one composition comprising an anion excess of chloride, phosphorus or sulfur.

In some aspects, the balance of metabolizable cations to metabolizable anions consumed by the animal can be adjusted by feeding the animal a composition having a stool quality altering amount of one or more metabolizable cations or metabolizable anions. Such compositions can include food compositions containing one or more ingredients suitable for consumption by an animal. In some aspects, the food composition comprises a dry food (i.e., a food containing from about 3 to about 11 % water). In other aspects, the food composition comprises a semi-moist food (i.e., a food containing from about 25 to about 35% water). In some aspects, the food composition comprises a moist food (i.e., a food containing from about 60 to more than about 87% water). In some aspects, the food composition comprises a treat, snack, supplement, or partially or fully edible toy.

In some aspects, the balance of metabolizable cations to metabolizable anions consumed by the animal can be adjusted in conjunction with the administration of one or more anti-diarrhea agents or anti-constipation agents. The term "anti-diarrhea agent" means any compound, composition, or drug useful for preventing or treating diarrhea. The term "anti-constipation agent" means any compound, composition, or drug useful for preventing or treating constipation.

Methods for Altering Nutrient Digestibility

It has surprisingly been discovered that protein digestion in a dog can be increased by feeding the dog a composition having a greater negative DCAB and that fat digestion in a dog can be increased by feeding a dog a composition having a greater positive DCAB. Typically desired target DCAB values range from about -200 to about -100 for optimum protein digestibility while a DCAB from about +70 to about +100 is desired to achieve good fat digestibility. The uses of the invention encompass the manipulation of the dietary cation anion balance in order to cause a modification in fat digestibility in a dog. In general, a dog food composition with a greater negative DCAB in comparison to another composition possesses greater protein digestibility and a dog food composition with a greater positive DCAB in comparison to another composition possesses greater fat digestibility.

In certain embodiments, an increase in fat digestibility may be desired under certain situations, for example, in an animal in which a gain in weight is desired, such as may be the case for a senior or geriatric dog, dogs with cancer or other underweight dogs. In addition, in certain embodiments of the description, increasing protein digestibility in a dog may be desired under certain situations, for example, in an animal suffering from irritable bowel disease or for reducing stool odor. It is further contemplated herein, however, that there are conditions in which a decrease in protein or fat digestibility in a dog may be desired. For example, reducing protein digestibility may be beneficial in a dog suffering from kidney disease. In other aspects, reducing fat digestibility may also be beneficial as a method for causing weight reduction in a dog. The methods described herein encompass not only modifying the DCAB of a composition fed to a dog to cause an increase in protein or fat digestibility but also, when therapeutically useful, to cause a reduction in protein or fat digestibility in a dog in need thereof. Furthermore, it is also contemplated herein that the methods described herein encompass the modification of both protein and fat digestibility in an animal when therapeutically beneficial, for example, by modifying the DCAB to be more negative, protein digestibility may be increased while fat digestibility may be decreased; by modifying the DCAB to be more positive, protein digestibility may be decreased while fat digestibility may be increased.

One of ordinary skill in the art will understand that there may be dogs in which an increase or decrease in either protein or fat digestibility is desired without the concomitant inverse change in digestibility of the other nutrient. For example, it may be desirable to modify DCAB to increase protein digestibility in a dog while minimizing any decrease in fat digestibility; conversely, it may be desirable to modify DCAB to increase fat digestibility without causing a significant decrease in protein digestibility. In certain aspects, it is possible that an increase in both protein and fat digestibility is desired. It is contemplated herein that in such cases of undesired inverse changes, one of skill in the art can supplement the dog's diet to either minimize the undesired decrease in nutrient digestibility or to cause a concomitant increase in digestibility, for example, by supplementing the diet with high quality, readily digestible forms of the nutrient the digestibility of which is to be maintained or increased. High quality, highly digestible sources of protein include egg, protein hydrolysates and isolates such as soy protein isolate and poultry liver hydrolysate. High quality, highly digestible sources of fat include soybean oil or fish oil. Similarly, where a decrease in digestibility of both protein and fat may be desired, it is contemplated herein that one of skill in the art may modify the DCAB of the composition fed to the dog to decrease the digestibility of one of the nutrients while also appropriately supplementing the diet of the dog with a less readily digestible form of the other nutrient. Examples of protein and fat that are less readily digestible in dogs include bone meal and tallow, respectively.

It is understood herein that with regard to an increase or decrease in digestibility of fat or protein, the actual positive or negative measure of DCAB of the diet to be fed is relative to what the animal has previously consumed. Thus, a DCAB that is more or less positive or negative, as the case may be, than the DCAB of what was previously fed, may be used in the methods described herein to achieve an increase or decrease in fat and/or protein digestibility in a dog as particularly described herein.

Methods for determining the digestibility of nutrients such as fat and protein are known in the art. In general, digestibility is determined by measuring the content ingested minus the content in the feces divided by the content ingested. Thus, in order to determine protein digestibility, one of skill in the art would determine the protein content of a composition and the protein content of stool produced by a dog fed the composition. The protein content of a composition may be determined by any number of methods known by those of skill in the art, for example, as published by the Association of Official Analytical Chemists in Official Methods of Analysis ("OMA").

Methods of Altering Food Intake

In another aspect, the description provides methods for altering food intake by an animal. The term "animal" includes any animal susceptible to or suffering from obesity as well as any animal in need of weight management including weight loss, weight maintenance or weight gain. An animal is "susceptible to" a disease or condition if the animal exhibits symptoms that indicate that the animal is likely to develop the condition or disease. An animal is "suffering from" a disease or condition if the animal exhibits symptoms that are indicative that the animal has developed the condition or disease.

In certain aspects, the animal is a member of the order Carnivora. In some such aspects, the animal is a canine, and in other such a feline. In some aspects, the animal is a companion animal. A companion animal can be, for example, an animal of any species that is kept as a pet. A companion animal can also be an animal from a variety of widely domesticated species, for example, dogs (Canis familiaris) and cats (Felis domesticus) regardless of whether or not the animal is kept solely as a pet. Thus, companion animals include, for example, working dogs, cats kept for rodent control, as well as pet cats and dogs.

The methods described herein include adjusting the balance of metabolizable cations to metabolizable anions regularly consumed by the animal by an amount effective to alter the animal's average food intake. Generally, the balance of metabolizable cations to metabolizable anions can be determined by any means known in the art. In one aspect, the metabolizable cations are selected from the group consisting of calcium, sodium, potassium and magnesium; and the metabolizable anions are selected from the group consisting of phosphorus, chloride and sulfur. One method for measuring the balance of metabolizable cations to metabolizable anions is to calculate the animal's dietary cation-anion balance (DCAB). DCAB is determined by calculating the cumulative amount of sodium, potassium, calcium, and magnesium regularly consumed by the animal and subtracting the cumulative amount of chloride, sulfur, and phosphorus regularly consumed by the animal. See, Baker et al., Comparative Nutrition of Cats and Dogs, Ann. Rev. Nutr. 11:239-63 (1991).

In adjusting the balance of metabolizable cations to metabolizable anions consumed by the animal, Applicants have discovered that increasing the balance of metabolizable cations to metabolizable anions will result in increased average food intake. Accordingly, decreasing the balance of metabolizable cations to metabolizable anions will result in decreased average food intake,

In certain aspects wherein the animal is in need of weight gain, for example, the animal is susceptible to or suffering from low body weight, food intake may be increased by increasing the balance of metabolizable cations to metabolizable anions. In such aspects, the balance can be increased by increasing the cumulative amount of calcium, sodium, potassium and magnesium regularly consumed by the animal relative to the cumulative amount of phosphorus, chloride and sulfur regularly consumed by the animal. For example, the balance of metabolizable cations to metabolizable anions may be increased by increasing the animal's dietary intake of at least one composition comprising calcium, sodium, potassium or magnesium. Likewise, the balance may be increased by decreasing the animal's dietary intake of at least one composition comprising chloride, phosphorus or sulfur.

In other aspects, wherein the animal is need of weight loss, for example, the animal is susceptible to or suffering from obesity, food intake can be decreased by decreasing the balance of metabolizable cations to metabolizable anions consumed by the animal. In such aspects, the balance can be decreased by decreasing the cumulative amount of calcium, sodium, potassium and magnesium regularly consumed by the animal relative to the cumulative amount of phosphorus, chloride and sulfur regularly consumed by the animal. For example, the balance of metabolizable cations to metabolizable anions can be decreased by decreasing the animal's dietary intake of at least one composition comprising calcium, sodium, potassium or magnesium. Likewise, the balance may be decreased by increasing the animal's dietary intake of at least one composition comprising chloride, phosphorus or sulfur.

In some aspects, the balance of metabolizable cations to metabolizable anions consumed by the animal can be adjusted by feeding the animal a composition comprising a food intake altering amount of one or more metabolizable cations or metabolizable anions. Such compositions can include food compositions. In some aspects, the food composition comprises a dry food (i.e., a food containing from about 3 to about 11% water). In other aspects, the food composition comprises a semi-moist food (i.e., a food containing from about 25 to about 35% water). In some aspects, the food composition comprises a moist food (i.e., a food containing from about 60 to more than about 87% water). In some aspects, the food composition comprises a treat, snack, supplement, or partially or fully edible toy.

In some aspects, the balance of metabolizable cations to metabolizable anions consumed by the animal can be adjusted in conjunction with the administration of one or more weight-loss agents. The term "weight-loss agent" means any compound, composition, or drug useful for regulating weight or preventing or treating obesity. Examples include, but are not limited to, commercially available "low fat" or "low calorie" pet food compositions. Likewise, in some aspects, the balance of metabolizable cations to metabolizable anions consumed by the animal can be adjusted in conjunction with the administration of one or more agents for promoting weight gain. The term "agent for promoting weight gain" means any compound, composition, or drug useful for increasing weight and may include, but is not limited to, commercial and/or prescription diet formulations available for nutritional supplementation.

In a further aspect, the present description provides for a use of a composition that comprises a food intake altering amount of at least one ingredient selected from the group consisting of metabolizable cations and metabolizable anions to prepare a medicament. In another, the invention provides for the use of such a composition to prepare a medicament for altering food intake. Generally, medicaments are prepared by admixing a compound or composition with excipients, buffers, binders, plasticizers, colorants, diluents, compressing agents, lubricants, flavorants, moistening agents, and other ingredients known to skilled artisans to be useful for producing medicaments and formulating medicaments that are suitable for administration to an animal.

EXAMPLES

This invention can be further illustrated by the following example. However, it should be understood that the example is included merely for purposes of illustration and is not intended to limit the scope of the invention unless otherwise specifically indicated.

Example 1

Seventy dogs were randomly assigned to one of 7 groups containing 10 dogs per group. Each group received one of 7 foods formulated to contain a different balance of metabolizable cations to metabolizable anions. The balance of metabolizable cations to metabolizable anions was calculated as DCAB described above comprising the cumulative amount of sodium, potassium, calcium, and magnesium minus the cumulative amount of chloride, sulfur and phosphorus. The dogs were fed the foods for 7 days over which time the dog's stools were collected and scored. Each stool sample was scored on a scale of 1 to 5 with 1 being watery and loose and 5 being ideal.

Results showing the nutrient analysis of each food, the average stool score for each food, and the number of stools scored for each food are presented in Table 1.

Table 1
Analyzed nutrient composition of foods fed to dogs[a]

| Item | Food 1 | Food 2 | Food 3 | Food 4 | Food 5 | Food 6 | Food 7 |
|---|---|---|---|---|---|---|---|
| Crude Protein (%) | 20.2 | 18.6 | 19.1 | 22.1 | 21.2 | 21.3 | 21.5 |
| Crude Fat (%) | 13.0 | 14.0 | 14.2 | 9.8 | 13.8 | 14.0 | 12.8 |
| Crude Fiber (%) | 2.9 | 3.0 | 3.2 | 1.4 | 4.1 | 4.7 | 3.7 |
| Sodium (%) | 0.39 | 0.29 | 0.30 | 0.30 | 0.43 | 0.43 | 0.39 |
| Potassium (%) | 0.87 | 0.64 | 0.71 | 0.63 | 1.29 | 1.55 | 1.52 |
| Chloride (%) | 0.97 | 0.77 | 0.74 | 0.72 | 0.83 | 0.79 | 0.76 |
| Sulfur (%) | 0.40 | 0.33 | 0.33 | 0.25 | 0.53 | 0.58 | 0.54 |
| Calcium (%) | 0.63 | 0.67 | 0.66 | 0.75 | 0.66 | 0.62 | 0.66 |
| Magnesium (%) | 0.06 | 0.05 | 0.05 | 0.16 | 0.09 | 0.11 | 0.11 |
| Phosphorus (%) | 0.58 | 0.55 | 0.50 | 0.75 | 0.58 | 0.63 | 0.62 |
| DCAB[b] (mEq) | -107 | -81 | -23 | +4 | +21 | +30 | +62 |
| Stool Score[c] | 4.0 | 4.1 | 4.3 | 4.3 | 4.6 | 4.8 | 4.6 |
| Stool Count[d] | 84 | 85 | 69 | 80 | 68 | 68 | 68 |

[a] Dry Matter Basis
[b] DCAB = (Sodium+Potassium+Calcium+Magnesium)-(Chloride+Sulfur+Phosphorous)
[c] Stool scores were ranked from 1 to 5 with 1 being watery and loose and 5 being ideal
[d] Number of stools scored over seven day period

The results show that the balance of metabolizable cations to metabolizable anions in the foods was directly related to dog stool quality and stool frequency. Dogs fed foods having a higher balance of metabolizable cations to metabolizable anions or DCAB demonstrated higher stool scores indicating improved stool quality and lower stool counts indicating a reduction in stool frequency.

Example 2

Three foods varying in DCAB (-128 mEq, +98 mEq and +167 mEq) are fed to six beagle dogs (average age 8.7 years) in order of increasing positive DCAB to determine the effect of base excess on nutrient digestibility. The ingredient and nutrient compositions of each food treatment are presented in Table 2. Each food is kibbled and formulated in accordance with AAFCO guidelines (e.g., Nutrient Guide for Dogs and Cats, 2006) and balanced to meet growing dog requirements. Each food is fed for a period of two weeks at maintenance levels (i.e., levels that maintain the weight of the animal) to the same group of six dogs and nutrient digestibility is measured for each food according to conventional methods. Stool quality is also assessed according to conventional methods. Data is provided in Table 3.

Table 2: Analyzed Nutrient Composition of Foods Fed to Dogs in Example 1 (dry matter basis)

| Nutrient | Diet with DCAB of -128 mEq | Diet with DCAB of +98 mEq | Diet with DCAB of +167 mEq |
|---|---|---|---|
| Crude Protein, % | 20.36 | 19.70 | 21.29 |
| Crude Fat, % | 14.05 | 15.57 | 14.30 |
| Ash, % | 5.30 | 4.50 | 3.72 |
| Crude Fiber, % | 3.77 | 3.58 | 3.85 |
| Potassium, % | 0.96 | 0.74 | 0.76 |
| Calcium, % | 0.88 | 0.80 | 0.74 |
| Sulfur, % | 0.73 | 0.22 | 0.27 |
| Chloride, % | 0.67 | 0.54 | 0.32 |
| Phosphorous, % | 0.60 | 0.65 | 0.55 |
| Sodium, % | 0.17 | 0.18 | 0.19 |
| Magnesium, % | 0.13 | 0.12 | 0.12 |

Table 3: Influence of Dietary Cation and Anion Balance on Nutrient Digestibility in Dogs in Example 1: Analyzed DCAB (mEq)

| | Diet -128 | Diet +98 | Diet +167 | SEM[a] |
|---|---|---|---|---|
| | | | | |
| Average Body Weight, kg | 13.4 | 13.0 | 13.0 | --- |
| Average Daily Intake, g | 263.5 | 268.6 | 273.1 | 17.9 |
| Dry Matter Digestibility, % | 83.23 | 82.48 | 83.57 | 1.39 |
| Crude Protein Digestibility, % | 84.35 | 80.04 | 81.16 | 1.59 |
| Fat Digestibility, % | 89.94 | 92.91 | 93.13 | 0.65 |
| Fiber Digestibility, % | 45.96 | 45.87 | 47.30 | 3.84 |
| Carbohydrate Digestibility, % | 92.91 | 94.03 | 94.71 | 0.75 |
| Energy Digestibility, % | 85.64 | 84.94 | 85.86 | 1.18 |
| Average Stool Rating | 3.43 | 3.37 | 3.48 | - |
| [a] Standard Error of Mean, n=6 | | | | |

Table 4: Influence of Dietary Cation and Anion Balance on Nutrient Digestibility in Dogs in Example 1: Comparison of Diets (probability of greater F- value)

| | -128 vs +98 | -128 vs +167 | +98 vs +167 |
|---|---|---|---|
| | | | |

(continued)

|  | -128 vs +98 | -128 vs +167 | +98 vs +167 |
|---|---|---|---|
| Average Body Weight, kg | -- | -- | -- |
| Average Daily Intake, g | 0.846 | 0.710 | 0.858 |
| Dry Matter Digestibility, % | 0.703 | 0.874 | 0.590 |
| Crude Protein Digestibility, % | 0.074 * | 0.176 | 0.627 |
| Fat Digestibility, % | 0.006* | 0.003* | 0.817 |
| Fiber Digestibility, % | 0.983 | 0.809 | 0.793 |
| Carbohydrate Digestibility, % | 0.302 | 0.105 | 0.520 |
| Energy Digestibility, % | 0.681 | 0.891 | 0.584 |
| * Indicates significant difference | | | |

Data from the feeding studies indicate that if the base excess is increased (DCAB is more negative) crude protein digestibility is increased. If base excess is decreased (DCAB more positive) fat digestibility is increased. Based on these data, it would appear that desired target DCAB values range from about -200 to about -100 to achieve an increase in protein digestibility while a DCAB from about +70 to about +100 is desired to achieve an increase in fat digestibility. Specifically, in these studies a DCAB of -128 is associated with an increase in protein digestibility while a DCAB of +98 is associated with an increase in fat digestibility. Interestingly, stool quality of the dogs in the feeding study did not appear to change significantly despite the modification of DCAB. The reason for this is unclear, although it may be due to the relatively advanced age of the dogs used in the study.

Example 3

Twenty dogs are randomly assigned to one of two groups containing 10 dogs per group. Each group receives one of two foods formulated to contain a different balance of metabolizable cations to metabolizable anions. The balance of metabolizable cations to metabolizable anions is calculated as DCAB according to conventional methods comprising the cumulative amount of sodium, potassium, calcium, and magnesium minus the cumulative amount of chloride, sulfur and phosphorus, taking into account the need to convert data for cations and anions from percentage to milliequivalents (mEq) as provided in Table 5. The dogs are fed the foods for seven days over which time the dog's average food intake is quantified.

Results showing the nutrient analysis of each food and the average food intake for each food are presented in Table 6.

Table 5

Analyzed Nutrient Composition of Foods Fed to Dogs[a]

| Item | Food 1 | Food 2 |
|---|---|---|
| Crude Protein (%) | 20.2 | 19.1 |
| Crude Fat (%) | 13.0 | 14.2 |
| Crude Fiber (%) | 2.9 | 3.2 |
| Sodium (%) | 0.39 | 0.30 |
| Potassium (%) | 0.87 | 0.71 |
| Chloride (%) | 0.97 | 0.74 |
| Sulfur (%) | 0.40 | 0.33 |
| Calcium (%) | 0.63 | 0.66 |
| Magnesium (%) | 0.06 | 0.05 |
| Phosphorus (%) | 0.58 | 0.50 |
| DCAB[b] (mEq) | -107 | -23 |
| Avg. food intake (g/day) | 152 | 194 |

[a] Dry Matter Basis
[b] DCAB = (Sodium+Potassium+Calcium+Magnesium)-(Chloride+Sulfur+Phosphorous)

Table 6

| Conversion Data for Calculating DCAB | | | | |
|---|---|---|---|---|
| Element | Molecular weight (g) | Valence | Equivalent weight (g) | To convert % to mEq multiply by this number |
| Sodium | 23.0 | 1 | 23.0 | 434.98 |
| Potassium | 39.1 | 1 | 39.1 | 255.74 |
| Chloride | 35.5 | 1 | 35.5 | 282.06 |
| Sulfur | 32.1 | 2 | 16.0 | 623.75 |
| Calcium | 40.1 | 2 | 20.0 | 499.00 |
| Magnesium | 24.3 | 2 | 12.2 | 822.64 |
| Phosphorous | 31.0 | 1.8 | 17.2 | 581.14 |

Results indicate that the balance of metabolizable cations to metabolizable anions in the foods is directly related to average food intake. Dogs fed Food 2 having a higher balance of metabolizable cations to metabolizable anions or DCAB demonstrate an average food intake increase of 42 g/day.

Unless defined otherwise, all technical and scientific terms and any acronyms used herein have the same meanings as commonly understood by one of ordinary skill in the art in the field of this invention. Although any compositions, methods, kits, and means for communicating information similar or equivalent to those described herein can be used to practice this invention, the preferred compositions, methods, kits, and means for communicating information are described herein.

**Claims**

1. Metabolizable cations and metabolizable anions for use in increasing fat digestibility in a dog in need thereof, wherein the metabolizable cations and metabolizable anions are provided in a composition, wherein the dog has a previous diet, wherein the dietary cation-anion balance (DCAB) of said composition is increased relative to the dietary cation-anion balance (DCAB) of the previous diet of the dog, and wherein the composition is administered to the dog.

2. Metabolizable cations and metabolizable anions for use according to claim 1, wherein said dog is a dog in which weight gain is desired.

3. Metabolizable cations and metabolizable anions for use in decreasing fat digestibility in a dog in need thereof, wherein the metabolizable cations and metabolizable anions are provided in a composition, wherein the dog has a previous diet, wherein the dietary cation-anion balance (DCAB) of said composition is decreased relative to the dietary cation-anion balance (DCAB) of the previous diet of the dog, and wherein the composition is administered to the dog.

4. Metabolizable cations and metabolizable anions for use according to claim 3, wherein said dog is a dog in which weight reduction is desired.

5. Metabolizable cations and metabolizable anions for use according to claim 3, wherein the use further comprises treating digestive problems in the dog.

6. Metabolizable cations and metabolizable anions for use according to claim 5, wherein the dietary cation-anion balance (DCAB) of said composition is decreased relative to dietary cation-anion balance (DCAB) of the previous diet of the dog by decreasing the amount of metabolizable cations for consumption by the dog, increasing the amount of metabolizable anions for consumption by the dog or by both decreasing the amount of metabolizable cations and increasing the amount of metabolizable anions for consumption by the dog.

7. Metabolizable cations and metabolizable anions for use according to claim 1, wherein the use further comprises treating digestive problems in the dog.

8. Metabolizable cations and metabolizable anions for use according to claim 7, wherein the dietary cation-anion balance (DCAB) of said composition is increased relative to the dietary cation-anion balance (DCAB) of the previous diet of the dog by increasing the amount of metabolizable cations for consumption by the dog, decreasing the amount of metabolizable anions for consumption by the dog or by both increasing the amount of metabolizable cations and decreasing the amount of metabolizable anions for consumption by the dog.

9. Use of metabolizable anions and metabolizable cations in the manufacture of a dog food composition to modify fat digestibility in a dog in need thereof,
wherein the dog has a previous diet,
wherein the dietary cation-anion balance (DCAB) of said composition is increased or decreased relative to the dietary cation-anion balance (DCAB) of the previous diet of the dog,
wherein the composition is administered to the dog, and
wherein a decrease in DCAB decreases fat digestibility, and an increase in DCAB increases fat digestibility in the dog.

**Patentansprüche**

1. Metabolisierbare Kationen und metabolisierbare Anionen zur Verwendung beim Erhöhen der Verdaulichkeit von Fett in einem Hund, der dessen bedarf,
wobei die metabolisierbaren Kationen und metabolisierbaren Anionen in einer Zusammensetzung bereitgestellt werden,
wobei der Hund eine vorherige Ernährung hat,
wobei die diätetische Kationen-Anionen-Bilanz (DCAB) der Zusammensetzung in Bezug auf die diätetische Kationen-Anionen-Bilanz (DCAB) der vorherigen Ernährung des Hundes erhöht wird und
wobei die Zusammensetzung dem Hund verabreicht wird.

2. Metabolisierbare Kationen und metabolisierbare Anionen zur Verwendung gemäß Anspruch 1, wobei der Hund ein Hund ist, bei welchem eine Gewichtszunahme gewünscht wird.

3. Metabolisierbare Kationen und metabolisierbare Anionen zur Verwendung beim Verringern der Verdaulichkeit von Fett in einem Hund, der dessen bedarf,
wobei die metabolisierbaren Kationen und metabolisierbaren Anionen in einer Zusammensetzung bereitgestellt werden,
wobei der Hund eine vorherige Ernährung hat,
wobei die diätetische Kationen-Anionen-Bilanz (DCAB) der Zusammensetzung in Bezug auf die diätetische Kationen-Anionen-Bilanz (DCAB) der vorherigen Ernährung des Hundes verringert wird und
wobei die Zusammensetzung dem Hund verabreicht wird.

4. Metabolisierbare Kationen und metabolisierbare Anionen zur Verwendung gemäß Anspruch 3, wobei der Hund ein Hund ist, bei welchem eine Gewichtsabnahme gewünscht wird.

5. Metabolisierbare Kationen und metabolisierbare Anionen zur Verwendung gemäß Anspruch 3, wobei die Verwendung weiterhin das Behandeln von Verdauungsproblemen bei dem Hund umfasst.

6. Metabolisierbare Kationen und metabolisierbare Anionen zur Verwendung gemäß Anspruch 5, wobei die diätetische Kationen-Anionen-Bilanz (DCAB) der Zusammensetzung in Bezug auf die diätetische Kationen-Anionen-Bilanz (DCAB) der vorherigen Ernährung des Hundes verringert wird, indem die Menge an metabolisierbaren Kationen zum Verzehr durch den Hund verringert wird, die Menge an metabolisierbaren Anionen zum Verzehr durch den Hund erhöht wird oder indem sowohl die Menge an metabolisierbaren Kationen verringert wird als auch die Menge an metabolisierbaren Anionen zum Verzehr durch den Hund erhöht wird.

7. Metabolisierbare Kationen und metabolisierbare Anionen zur Verwendung gemäß Anspruch 1, wobei die Verwendung weiterhin das Behandeln von Verdauungsproblemen bei dem Hund umfasst.

8. Metabolisierbare Kationen und metabolisierbare Anionen zur Verwendung gemäß Anspruch 7, wobei die diätetische Kationen-Anionen-Bilanz (DCAB) der Zusammensetzung in Bezug auf die diätetische Kationen-Anionen-Bilanz (DCAB) der vorherigen Ernährung des Hundes erhöht wird, indem die Menge an metabolisierbaren Kationen zum Verzehr durch den Hund erhöht wird, die Menge an metabolisierbaren Anionen zum Verzehr durch den Hund verringert wird oder indem sowohl die Menge an metabolisierbaren Kationen erhöht wird als auch die Menge an metabolisierbaren Anionen zum Verzehr durch den Hund verringert wird.

9. Verwendung von metabolisierbaren Anionen und metabolisierbaren Kationen bei der Herstellung einer Nahrungsmittelzusammensetzung für einen Hund, um die Verdaulichkeit von Fett in einem Hund, der dessen bedarf, zu modifizieren,
wobei der Hund eine vorherige Ernährung hat,
wobei die diätetische Kationen-Anionen-Bilanz (DCAB) der Zusammensetzung in Bezug auf die diätetische Kationen-Anionen-Bilanz (DCAB) der vorherigen Ernährung des Hundes erhöht oder verringert wird,
wobei die Zusammensetzung dem Hund verabreicht wird und
wobei eine Abnahme bei der DCAB die Verdaulichkeit von Fett verringert und eine Zunahme bei der DCAB die Verdaulichkeit von Fett bei dem Hund erhöht.

## Revendications

1. Cations métabolisables et anions métabolisables pour une utilisation dans l'augmentation de la digestibilité des graisses chez un chien en ayant besoin.
dans lesquels les cations métabolisables et les anions métabolisables sont fournis dans une composition,
dans lesquels le chien a un régime alimentaire précédent,
dans lesquels l'équilibre cation-anion alimentaire (DCAB) de ladite composition est augmenté par rapport à l'équilibre cation-anion alimentaire (DCAB) du régime alimentaire précédent du chien, et
dans lesquels la composition est administrée au chien.

2. Cations métabolisables et anions métabolisables pour une utilisation selon la revendication 1, dans lesquels ledit chien est un chien chez lequel une prise de poids est souhaitée.

3. Cations métabolisables et anions métabolisables pour une utilisation dans la diminution de la digestibilité des graisses chez un chien en ayant besoin.
dans lesquels les cations métabolisables et les anions métabolisables sont fournis dans une composition,
dans lesquels le chien a un régime précédent,
dans lesquels l'équilibre cation-anion alimentaire (DCAB) de ladite composition est diminué par rapport à l'équilibre cation-anion alimentaire (DCAB) du régime alimentaire précédent du chien, et
dans lesquels la composition est administrée au chien.

4. Cations métabolisables et anions métabolisables pour une utilisation selon la revendication 3, dans lesquels ledit chien est un chien chez lequel une réduction de poids est souhaitée.

5. Cations métabolisables et anions métabolisables pour une utilisation selon la revendication 3, dans lesquels l'utilisation comprend en outre le traitement de problèmes digestifs chez le chien.

6. Cations métabolisables et anions métabolisables pour une utilisation selon la revendication 5, dans lesquels l'équilibre cation-anion alimentaire (DCAB) de ladite composition est diminué par rapport à l'équilibre cation-anion alimentaire (DCAB) du régime alimentaire précédent du chien par diminution de la quantité de cations métabolisables à consommer par le chien, augmentation de la quantité d'anions métabolisables à consommer par le chien ou à la fois diminution de la quantité de cations métabolisables et augmentation de la quantité d'anions métabolisables à consommer par le chien.

7. Cations métabolisables et anions métabolisables pour une utilisation selon la revendication 1, dans lesquels l'utilisation comprend en outre le traitement de problèmes digestifs chez le chien.

8. Cations métabolisables et anions métabolisables pour une utilisation selon la revendication 7, dans lesquels l'équilibre cation-anion alimentaire (DCAB) de ladite composition est augmenté par rapport à l'équilibre cation-anion alimentaire (DCAB) du régime alimentaire précédent du chien par augmentation de la quantité de cations métabolisables à consommer par le chien, diminution de la quantité d'anions métabolisables à consommer par le chien ou

**EP 2 352 387 B1**

à la fois augmentation de la quantité de cations métabolisables et diminution de la quantité d'anions métabolisables à consommer par le chien.

9. Utilisation d'anions métabolisables et de cations métabolisables dans la fabrication d'une composition d'aliment pour chien afin de modifier la digestibilité des graisses chez un chien en ayant besoin,
dans laquelle le chien a un régime alimentaire précédent,
dans laquelle l'équilibre cation-anion alimentaire (DCAB) de ladite composition est augmenté ou diminué par rapport à l'équilibre cation-anion alimentaire (DCAB) du régime alimentaire précédent du chien,
dans laquelle la composition est administrée au chien, et
dans laquelle une diminution du DCAB diminue la digestibilité des graisses, et une augmentation du DCAB augmente la digestibilité des graisses chez le chien.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 56651206 A
- US 74163205 P
- US 62011907 A
- US 76534106 P
- WO 2007065172 A1
- US 6517877 B
- US 6280779 B
- US 5616569 A

### Non-patent literature cited in the description

- Nutrient Requirements of Swine. Academy Press, 1998
- **DERJANT-LI et al.** *J. Anim. Sci.,* 2001, vol. 79, 1840-1848
- **HAYDON ; WEST.** *J. Anim. Sci,* 1990, vol. 68, 3687-3693
- **MAHAN et al.** *J. Anim. Sci.,* 1999, vol. 77, 3016-3021
- **MEYER H. et al.** *J. Vet. Med.,* 1999, vol. 46, 155-165
- **DERSJANT-LI ; 2001 et al.** *J. Anim. Physiol. a. Anim. Nutr.,* vol. 85, 101-109
- **BAKER.** *Comparative Nutrition of Cats and Dogs, Ann. Rev. Nutr.,* 1991, vol. 11, 239-63
- *Nutrient Requirements of Dogs and Cats,* 2006
- **BAKER ; CZARNECKI-MAULDEN.** *Annu. Rev. Nutr.,* 1991, vol. 11, 239-263
- **SUNVOLD et al.** *J Anim Sci,* 1995, vol. 73, 1099-1109
- **YAMKA et al.** *Am J Vet Res,* 2006, vol. 67 (1), 88-94
- **BAKER et al.** Comparative Nutrition of Cats and Dogs. *Ann. Rev. Nutr.,* 1991, vol. 11, 239-63
- *Nutrient Guide for Dogs and Cats,* 2006